Europäisches Patentamt

European Patent Office

Office européen des brevets

(19)

(11) Publication number: **0 068 603**
**B1**

# (12) EUROPEAN PATENT SPECIFICATION

(45) Date of publication of patent specification: **04.12.85**

(21) Application number: **82302012.8**

(22) Date of filing: **20.04.82**

(51) Int. Cl.⁴: **B 01 J 21/06, B 01 J 23/06,**
**B 01 J 37/02, C 07 C 1/04,**
**C 07 C 1/20, B 01 J 21/04,**
**C 07 C 1/02, C 10 G 3/00,**
**B 01 J 21/12, B 01 J 21/14**

(54) **Amorphous silica-based catalyst and process for its production.**

(30) Priority: **19.06.81 GB 8118956**

(43) Date of publication of application:
**05.01.83 Bulletin 83/01**

(45) Publication of the grant of the patent:
**04.12.85 Bulletin 85/49**

(84) Designated Contracting States:
**BE DE FR NL SE**

(56) References cited:
**EP-A-0 039 996**
**DE-A-2 102 597**
**FR-A-2 268 771**
**FR-A-2 372 770**
**FR-A-2 388 782**
**FR-A-2 454 328**
**GB-A-1 028 166**
**US-A-4 009 199**
**US-A-4 072 733**

(73) Proprietor: **Coal Industry (Patents) Limited**
**Hobart House Grosvenor Place**
**London SW1X 7AE (GB)**

(72) Inventor: **Robinson, Joseph Gordon**
**'Claybrook' The Hyde**
**Winchcombe Gloucestershire (GB)**
Inventor: **Barnes, David Ian**
**70 Fiddlers Green Lane**
**Cheltenham Gloucestershire (GB)**
Inventor: **Carswell, Angela Mary**
**21 The Willows**
**Longhope Gloucestershire (GB)**

(74) Representative: **Wood, John Irwin**
**Hobart House Grosvenor Place**
**London SW1X 7AE (GB)**

Courier Press, Leamington Spa, England.

## Description

This invention relates to an amorphous silica based catalyst and to a process for its production. The catalyst will find particular but not exclusive use in the conversion of synthesis gas, methanol or ethanol to hydrocarbons.

As the world's reserves of oil are being exhausted, much attention is being focused on the use of coal as a feedstock for the preparation of substitutes for oil-based products. Processes are known whereby coal can be treated with steam and oxygen at elevated temperatures to produce synthesis gas, comprising predominantly hydrogen and carbon monoxide. Synthesis gas may be converted by known reactions into methanol. It is desirable to be able to convert synthesis gas or methanol into higher hydrocarbons which can be used as starting materials in the preparation of substitutes for oil-based products.

It has been proposed in US Patent No. 4,009,199 to produce catalysts especially suitable for the esterification of dimethyl terephthalate by impregnating silica gel with a solution of an aluminium, titanium, zinc or tin compound, separating the excess solution and drying at elevated temperature to remove the solvent. Preferably, the catalyst is treated with a methanol/steam mixture at 150°C before use.

It is an object of the present invention to provide a catalyst which can at least in part convert synthesis gas or methanol into a mixture of hydrocarbons.

According to the present invention a catalyst for synthesis gas or methanol conversion comprises a highly porous amorphous silica having a monolayer of an amphoteric metal chemically bonded onto up to 90% of the surface of the silica matrix, the catalyst having a maximum pore diameter of about 1.5 nm.

Since the amphoteric metal will have a valence of 2 or 3, the surface of the silica will not be electrically neutral unless balancing ionic species are present. Preferably, the balancing ionic species are protons, although other cations may be used, either alone or in any combination with or without protons. Suitable other cations include transition metal cations, lanthanide cations and ammonium ions.

Preferably, the amphoteric metal is aluminium, although metals such as zinc, magnesium, zirconium or titanium may also be used.

Conveniently, only about 50% of the surface of the matrix has the monolayer bonded onto it and preferably the maximum pore diameter is about 0.5 nm. The catalyst may further include a monolayer of hydroxylated silicon atoms deposited on the highly porous amorphous silica. The monolayer of amphoteric metal is then bonded onto the monolayer of silicon atoms. In this way it is possible to alter the maximum pore diameter of the catalyst while retaining the monolayer of amphoteric metal chemically bonded onto the silica matrix.

Amorphous silicas are known and are usually available in hydroxylated form as silica gels (often known as xerogels). In amorphous silicas the silicon atoms are tetrahedrally co-ordinated in a matrix of oxygen atoms. In xerogel form the surface of the silica is provided predominantly with hydroxyl groups. Amorphous silicas are easily prepared and available in a wide variety of particle sizes and pore diameters. Usually most of the surface area of the gel is constituted by the pore walls. They are generally used as chromatography media and do not exhibit any significant catalytic activity in the conversion of synthesis gas or methanol to hydrocarbons.

It is thought that in the catalyst of the present invention the amphoteric metal atom bonded as a monolayer onto the silica matrix is constrained to adopt tetrahedral co-ordination. Since the metal will be di- or tri-valent, the surface will need to have two or one monovalent cations respectively associated with it in order to balance the electrical charges on the surface. When the cation is a proton, this will give rise to strongly acid sites on the catalyst surface. These are thought to be responsible for the catalytic activity. Since the catalytic activity is thought to be centred on a proton it is likely that the products of catalytic reactions will include aromatic rings and branched chain alkanes formed via carbonium ion intermediates. Although the applicants believe that this is the rationale behind the action of the catalyst, they do not wish to be limited by such explanation.

The pore diameter of the catalyst determines to a certain extent the product type and distribution. At a maximum pore diameter of about 1.5 nm the pores can accommodate mononuclear polysubstituted aromatics up to about $(C_{14})$. However, with maximum pore diameter of 0.5 nm the largest molcules which can be accommodated are approximately the size of durene $(C_{10})$.

If it is desired to convert synthesis gas directly to hydrocarbons, the catalyst of the present invention should be mixed with a catalyst impregnated with a transition metal, such as iron, to convert the synthesis gas in situ to Fischer-Tropsch type products by reduction of the carbon monoxide.

Alternatively, the second catalyst may be impregnated with zinc and chromium or zinc and copper compounds to convert the synthesis gas to methanol. Other second catalysts which may be used to produce suitable intermediates for conversion to hydrocarbons by the catalyst according to the invention include an alumina or silica support impregnated with lanthanide metals such as ruthenium.

The present invention also includes a method of preparing the catalyst described above. Therefore according to a second aspect of the invention, a process for the production of a catalyst for synthesis gas or methanol conversion comprises treating a highly porous amorphous silica with a solution of a hydrolysable compound of an amphoteric metal, removing the solvent to leave a monolayer of the compound on up to 90% of the surface area of the silica matrix, and causing the surface of the silica to react with the compound to produce a catalyst having a monolayer of the amphoteric metal chemically bonded onto up to 90% of the surface of the silica matrix and a maximum pore diameter of about 1.5 nm.

Any parts of the hydrolysable compound which have not reacted with the silica matrix should subsequently be hydrolysed by reaction with water.

The catalyst may subsequently be heated to dehydrate the surface layer to produce complete reaction of the amphoteric metal with the silica matrix so that anionic sites are created on the surface of the catalyst.

The compound may be an alkoxide or halide of any one of the metals mentioned above. Preferably the compound is a chloride or secondary butoxide. The solvent may be any relatively low boiling liquid in which the compound may be dissolved without hydrolysis occurring. Suitable liquids include the lower alkanes, and in particular hexane.

Preferably, the silica is dried before it is treated with the solution of the hydrolysable compound so that any physically absorbed water thereon is removed before it can react with the compound.

Conveniently, the highly porous silica has a mean pore diameter of about 2 nm, in which case the presence of the monolayer of chemically bonded amphoteric metal will reduce the pore diameter by the necessary amount. If the silica has a larger pore diameter, or if a smaller pore diameter is required, the process should include the step of depositing one or more layers of silica on the pore surface area. This may be done by depositing a hydrolysable silicon compound such as silicon tetrachloride on the highly porous silica and hydrolysing it to produce the required pore diameter.

A mixed catalyst for the conversion of synthesis gas to methanol may be prepared by mixing the catalyst prepared as described above with a suitable second catalyst, for instance, any one of the types of catalyst described above.

It is envisaged that the catalyst according to the invention will be of particular but not exclusive use in the conversion of synthesis gas or methanol into mixtures of branched and aromatic hydrocarbons, although it will also be of use in the conversion of higher alcohols such as ethanol or mixtures thereof into hydrocarbons. The catalyst may be used in any of the known catalytic reactors for the conversion of gases or liquids. The catalyst may be present as a fixed, moving or fluidised bed.

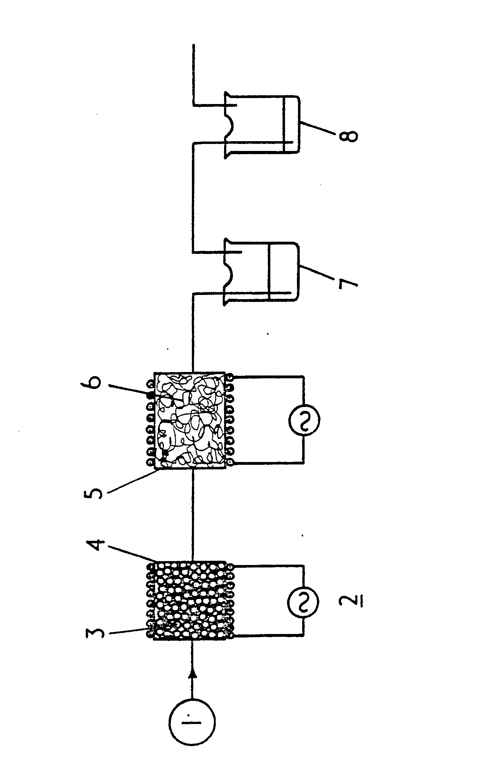

The invention will now be described by way of example only with reference to the accompanying figure which shows diagrammatically a part of a synthesis gas conversion plant.

Catalyst Preparation

Beads (100 g) of a highly porous amorphous silica gel having a mean pore diameter of 2 nm, a pore surface area of 800 m²/g, a pore volume of 0.4 cm³/g and a particle size of 125 microns were heated at 150°C for two hours in a dry atmosphere to remove any moisture physically absorbed onto the beads. The dried beads were placed under a slight vacuum.

A solution of aluminium butoxide in dry hexane (40 ml, 1.4 M) was cooled and added to the beads. The mixture was gently shaken. The shaking and vacuum were applied in order to ensure that all air in the pores was removed and that the solution was able to penetrate completely into the pores. After the vacuum was released, the solvent was removed from the beads over a period of about 2 hours by evaporation. The desolvated beads were allowed to stand at room temperature for about 16 hours. During this time it was assumed that the hydroxyl groups on the silica surface reacted with the aluminium compound, hydrolysing some of the aluminium-butoxide groups to form a monolayer of alkoxylated aluminium on the silica surface. Secondary butanol was evolved during the reaction. The amount of aluminium secondary butoxide in the solution was selected so that only 50% of the surface area of the silica was covered by alkoxylated aluminium. The beads were then added to an excess of water under slight vacuum so that the water penetrated the pores completely. The mixture was left to stand at room temperature so that the remaining butoxide groups were hydrolysed to hydroxyl groups. The beads were washed free of the secondary butanol formed during the hydrolysis and were dried by heating at 150°C. The catalyst thus formed had a maximum pore diameter of 1.5 nm.

The catalyst was divided into three portions. One portion was not further treated, and the other two portions were heated to 400°C and 500°C respectively.

A second type of catalyst was prepared from a similar silica gel. However, as a preliminary step beads (100 g) of the xerogel were dried at 120°C at a pressure of 10 mm Hg for 4 hours to remove any absorbed surface moisture. The beads were allowed to cool under an atmosphere of dry nitrogen. When the beads had cooled to room temperature an excess of silicon tetrachloride was added to them and the pressure reduced to ensure that the silicon tetrachloride completely filled the pores of the beads. The beads saturated with silicon tetrachloride were allowed to stand at room temperature for 24 hours. During this time surface hydroxyl groups on the xerogel reacted to hydrolyse at least some of the silicon-chlorine bonds, thereby bonding a monolayer of silicon atoms onto the xerogel surface.

Excess silicon tetrachloride was evaporated off by heating the beads to 50°C and applying vacuum. Distilled water was then added to the flask and vacuum applied to ensure that the water completely penetrated the pores of the xerogel. The water hydrolysed the remaining silicon-chlorine bonds to produce a monolayer of hydrated silicon bond onto the surface of the xerogel.

The hydrated zerogel thus produced was heated at 120° under reduced pressure for 4 hours and at 250°C for a further 4 hours. This produced a treated xerogel having a smaller pore diameter than the original.

The treated silica gel was then reacted with a solution of aluminium butoxide exactly as described above, and only differed from it in that it produced a catalyst having a maximum pore diameter of 0.5 nm. The catalyst was also divided into three portions and was heat treated as described above.

Each sample of catalyst was then used to convert methanol to hydrocarbons in the apparatus shown in the Figure, to which reference is now made.

Methanol was produced from synthesis gas in a reactor 1 and supplied to a heating chamber 2 comprising a bed of non-porous glass beads 3 in an electrically heated container 4. The glass beads 3 were maintained at 370°C. The heated vapour was passed from the chamber 2 to reaction vessel 5 which contained the catalyst 6. The catalyst 6 was maintained at a temperature of 375°C and the vapour was passed through the vessel 5 at a LHSV of 1. The products of the reaction were collected in traps 7 and 8 and analysed. Trap 8 was cooled in liquid nitrogen to enable gaseous products to be collected.

The results of the analysis showed that in all cases about 50% of the methanol was converted into hydrocarbons. These were mainly branched chain alkanes and substituted benzenes.

The same procedure was followed, except that the LHSV was 0.3 and the temperature of the catalyst was 450°C. In this case conversion of up to 95% of the methanol was achieved. About 30% of the products were aromatics.

The product distributions for the catalysts of maximum pore diameter 1.5 nm were very similar and did not appear to depend to any great extent on the heat treatment. However there was a sharp cut off at $C_{14}$ molecules. Similarly the 0.5 nm catalysts gave similar product ranges in all three cases, but the cut off point was at $C_{10}$ molecules.

In preliminary experiments, the catalysts prepared above were mixed intimately with a commercially available conventional Fischer-Tropsch catalyst. Each mixture of catalysts was heated to 450°C and synthesis gas was passed through at a LHSV of about 0.3. Significant amounts of hydrocarbons, including aromatics were collected. It was assumed that some paraffinic hydrocarbons were produced from the synthesis gas by the Fischer-Tropsch reaction. However, it was apparent that oxycarbon compounds produced by the Fischer-Tropsch reaction were converted to hydrocarbons by the catalyst according to the invention. Moreover, it appeared that some of the paraffinic hydrocarbons were cyclised by the catalyst according to the invention.

Thus the present invention provides a novel catalyst which can be used alone to convert methanol and other lower alkanols to hydrocarbons. Moreover, it can be used in combination with other catalysts to convert synthesis gas to hydrocarbons.

Claims

1. A catalyst for synthesis gas or methanol conversion, comprising a highly porous amorphous silica having a layer of an amphoteric metal chemically bonded onto the surface area of the silica matrix, characterised in that, a monolayer of an amphoteric metal covers up to 90% of the surface area of the silica matrix and that the catalyst has a maximum pore diameter of about 1.5 nm.

2. A catalyst according to claim 1, characterised in that the metal is selected from the group consisting of zinc, magnesium, zirconium and titanium.

3. A catalyst according to claim 1, characterised in that the metal is aluminium.

4. A catalyst according to any one of the preceding claims, characterised in that the monolayer covers up to 50% of the surface of the silica matrix.

5. A catalyst according to any one of the preceding claims, characterised in that electrical neutrality of the catalyst is maintained by the presence of protons on the surface of the matrix.

6. A catalyst according to any one of the preceding claims, characterised in that it includes a monolayer of hydroxylated silicon atoms bonded onto the silica matrix, the amphoteric metal being bonded onto the monolayer of silicon atoms.

7. A catalyst according to claim 6, characterised in that the maximum pore diameter is 0.5 nm.

8. A catalyst according to any one of the preceding claims, characterised in that a second catalyst for synthesis gas conversion is intimately mixed therewith.

9. A process for the production of a catalyst for synthesis gas or methanol conversion wherein a highly porous amorphous silica is treated with a solution of a hydrolysable compound of an amphoteric metal, the solvent is removed to leave a layer of the compound on the surface area of the silica matrix, and the surface of the silica is caused to react with the compound, characterised in that the solvent is removed to leave a monolayer of the compound on up to 90% of the surface area of the silica matrix and the catalyst produced has a maximum pore diameter of about 1.5 nm.

10. A process according to claim 9, characterised in that the catalyst is heated, to dehydrate the surface layer.

11. A process according to either one of claims 9 and 10, characterised in that the compound is an alkoxide or halide of the amphoteric metal.

12. A process according to any one of claims 9 to 11, characterised in that a monolayer of hydroxylated silicon atoms is chemically bonded onto the surface of the silica matrix prior to treating the silica with the hydrolysable compound.

13. A process according to any one of claims 9 to 12, characterised in that the catalyst is intimately mixed with a second catalyst capable of converting synthesis gas to oxycarbon products.

14. A process of converting a lower alkanol to hydrocarbons comprising passing the lower alkanol as a vapour at between 375 and 450°C and a LHSV of up to about 1 over a catalyst according to any one of claims 1 to 7 or made according to the process of any one of claims 9 to 12.

15. A process of converting synthesis gas to hydrocarbons comprising passing it at between 375 and 450°C and a LHSV of up to about 1 over a catalyst according to claim 8 or made by a process according to claim 13.

## Revendications

1. Catalyseur pour la conversion du gaz de synthèse ou du méthanol, comprenant une silice amorphe hautement poreuse ayant une couche d'un métal amphotère chimiquement fixée à la surface de contact de la matrice de silice, catalyseur caractérisé en ce qu'une monocouche du métal amphotère couvre jusqu'à 90% de la surface de contact de la matrice de silice et en ce que le catalyseur possède un diamètre maximal des pores d'environ 1,5 nm.

2. Catalyseur selon la revendication 1, caractérisé en ce que le métal est choisi dans l'ensemble constitué par le zinc, le magnésium, le zirconium et le titane.

3. Catalyseur selon la revendication 1, caractérisé en ce que le métal est l'aluminium.

4. Catalyseur selon l'une quelconque des revendications précédentes, caractérisé en ce que la monocouche couvre jusqu'à 50% de la surface de la matrice de silice.

5. Catalyseur selon l'une quelconque des revendications précédentes, caractérisé en ce qu'une neutralité catalytique du catalyseur est maintenue par la présence de protons à la surface de la matrice.

6. Catalyseur selon l'une quelconque des revendications précédentes, caractérisé en ce qu'il inclut une monocouche d'atomes de silicium hydroxylé fixée à la matrice de silice, le métal amphotère étant fixé sur la monocouche des atomes de silicium.

7. Catalyseur selon la revendication 6, caractérisé en ce que le diamètre maximal des pores est de 0,5 nm.

8. Catalyseur selon l'une quelconque des revendications précédentes, caractérisé en ce qu'un second catalyseur pour la conversion du gaz de synthèse est intimement mélangé à ce catalyseur.

9. Procédé de préparation d'un catalyseur pour la conversion du gaz de synthèse ou du méthanol, selon lequel on soumet une silice amorphe hautement poreuse à un traitement par une solution d'un composé hydrolysable d'un métal amphotère, on enlève le solvant pour laisser une couche du composé à la surface de contact de la matrice de silice et l'on provoque la réaction de la surface de la silice avec le composé, procédé caractérisé en ce qu'on enlève le solvant pour laisser une monocouche de composé sur jusqu'à 90% de la surface de contact de la matrice de silice, et en ce que le catalyseur produit a un diamètre maximal des pores d'environ 1,5 nm.

10. Procédé selon la revendication 9, caractérisé en ce qu'on chauffe le catalyseur pour déshydrater la couche de surface.

11. Procédé selon l'une des revendications 9 et 10, caractérisé en ce que le composé est un alcoolate ou halogénure du métal amphotère.

12. Procédé selon l'une quelconque des revendications 9 à 11, caractérisé en ce qu'on fixe chimiquement, à la surface de la matrice de silice, une monocouche d'atomes de silicium hydroxylé avant de traiter la silice par le composé hydrolysable.

13. Procédé selon l'une quelconque des revendications 9 à 12, caractérisé en ce qu'on mélange intimement le catalyseur avec un second catalyseur capable de convertir le gaz de synthèse en des produits oxycarbonés.

14. Procédé pour convertir un alcanol inférieur en des hydrocarbures, comprenant le passage de l'alcanol inférieur, sous forme de vapeur, à une température comprise entre 375 et 450°C et à une vitesse spatiale horaire (VVH) allant jusqu'à 1 environ sur un catalyseur selon l'une quelconque des revendications 1 à 7 ou préparé selon le procédé de l'une quelconque des revendications 9 à 12.

15. Procédé pour convertir du gaz de synthèse en des hydrocarbures, comprenant le passage du gaz, à une température comprise entre 375° et 450°C et à une vitesse spatiale horaire allant jusqu'à 1 environ, sur un catalyseur selon la revendication 8 ou préparé par un procédé selon la revendication 13.

## Patentansprüche

1. Katalysator zur Synthesegas- oder Methanol-Umwandlung, der hoch poröses amorphes Siliciumdioxid umfaßt, das eine Schicht aus einem amphoteren Metall aufweist, das chemisch an die Oberfläche der Siliciumdioxidmatrix gebunden ist, dadurch gekennzeichnet, daß eine Monoschicht eines amphoteren Metalls bis zu 90% der Oberfläche der Siliciumdioxidmatrix bedeckt und daß der Katalysator einen maximalen Porendurchmesser von etwa 1,5 nm aufweist.

2. Katalysator nach Anspruch 1, dadurch gekennzeichnet, daß das Metall aus einer Gruppe ausgewählt wird, die aus Zink, Magnesium, Zirkonium und Titan besteht.

3. Katalysator nach Anspruch 1, dadurch gekennzeichnet, daß das Metall Aluminium ist.

4. Katalysator nach einem der vorstehenden Ansprüche, dadurch gekennzeichnet, daß die Monoschicht bis zu 50% der Oberfläche der Siliciumdioxidmatrix bedeckt.

5. Katalysator nach einem der vorstehenden Ansprüche, dadurch gekennzeichnet, daß die elektrische Neutralität des Katalysators durch die Gegenwart von Protonen an der Oberfläche der Matrix aufrechterhalten wird.

6. Katalysator nach einem der vorstehenden Ansprüche, dadurch gekennzeichnet, daß er eine Monoschicht von hydroxilierten Siliciumatomen aufweist, die an die Siliciumdioxidmatrix gebunden sind, wobei das amphotere Metall an die Monoschicht der Siliciumatome gebunden ist.

7. Katalysator nach Anspruch 6, dadurch gekennzeichnet, daß der maximale Porendurchmesser 0,5 nm beträgt.

8. Katalysator nach einem der vorstehenden Ansprüche, dadurch gekennzeichnet, daß ein zweiter Katalysator zur Synthesegasumwandlung damit innig vermischt ist.

9. Verfahren zur Herstellung eines Katalysators zur Synthesegas- oder Methanolumwandlung, bei dem hoch poröses amorphes Siliciumdioxid mit einer Lösung einer hydrolisierbaren Verbindung eines amphoteren Metalls behandelt wird, das Lösungsmittel entfernt wird, um eine Schicht der Verbindung auf der Oberfläche der Siliciumdioxidmatrix zurückzulassen und die Oberfläche des Siliciumdioxids veranlaßt wird, mit der Verbindung zu reagieren, dadurch gekennzeichnet, daß das Lösungsmittel entfernt wird, um eine Monoschicht der Verbindung auf bis zu 90% der Oberfläche der Siliciumdioxidmatrix zurückzulassen und der hergestellte Katalysator einen maximalen Porendurchmesser von etwa 1,5 nm aufweist.

10. Verfahren nach Anspruch 9, dadurch gekennzeichnet, daß der Katalysator erwärmt wird, um die Oberflächenschicht zu dehydratisieren.

11. Verfahren nach einem der Ansprüche 9 und 10, dadurch gekennzeichnet, daß die Verbindung ein Alkoxid oder Halogenid des amphoteren Metalls ist.

12. Verfahren nach einem der Ansprüche 9 bis 11, dadurch gekennzeichnet, daß eine Monoschicht hydroxilierter Siliciumatome an die Oberfläche der Siliciumdioxidmatrix chemisch gebunden ist, bevor das Siliciumdioxid mit der hydrolisierbaren Verbindung behandelt wird.

13. Verfahren nach einem der Ansprüche 9 bis 12, dadurch gekennzeichnet, daß der Katalysator innig mit einem zweiten Katalysator vermischt wird, der in der Lage ist, Synthesegas zu Sauerstoff-Kohlenstoff-Produkten umzuwandeln.

14. Verfahren zur Umwandlung eines niedrigen Alkanols zu Kohlenwasserstoffen, bei dem das niedrige Alkanol als Dampf zwischen 375 und 450°C und bei einem Raumvolumendurchsatz (LHSV) bis zu etwa 1 über einen Katalysator nach einem der Ansprüche 1 bis 7 oder über einen Katalysator geleitet wird, der nach dem Verfahren nach einem der Ansprüche 9 bis 12 hergestellt ist.

15. Verfahren zur Umwandlung von Synthesegas zu Kohlenwasserstoffen, bei dem dasselbe zwischen 375 und 450°C und bei einem Raumvolumendurchsatz (LHSV) bis zu etwa 1 über einen Katalysator nach Anspruch 8 oder über einen Katalysator geleitet wird, der nach dem Verfahren gemäß dem Anspruch 13 hergestellt ist.